(19) Europäisches Patentamt
European Patent Office
Office européen des brevets

(11) Veröffentlichungsnummer : **0 507 763 A1**

(12) # EUROPÄISCHE PATENTANMELDUNG

(21) Anmeldenummer : **92890075.2**

(51) Int. Cl.[5] : **C07D 409/12,** A61K 31/41

(22) Anmeldetag : **01.04.92**

(30) Priorität : **04.04.91 AT 716/91**

(43) Veröffentlichungstag der Anmeldung : **07.10.92 Patentblatt 92/41**

(84) Benannte Vertragsstaaten : **PT**

(71) Anmelder : **Laevosan-Gesellschaft m.b.H.**
**Estermannstrasse 17**
**A-4020 Linz (AT)**

(72) Erfinder : **Binder, Dieter Prof. Dr.**
**Sieveringerstrasse 207**
**A-1190 Wien (AT)**
Erfinder : **Greier, Gerhard DR.**
**Estermannstrasse 17**
**A-4020 Linz (AT)**

(74) Vertreter : **Pawloy, Heinrich, Dr. et al**
**Riemergasse 14**
**A-1010 Wien (AT)**

(54) **Neue Thiophen-2-carbonsäurederivate und Verfahren zu deren Herstellung.**

(57) Beschrieben werden neue Verbindungen der allgemeinen Formel (I)

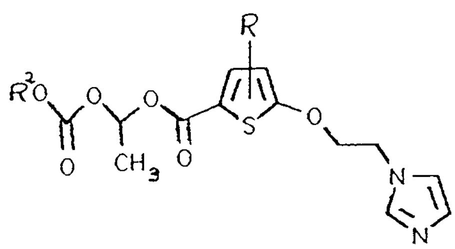

(I) ,

worin R in Stellung 3 oder 4 Wasserstoff, Methyl, Chlor oder Brom darstellt und $R^2$ $C_1$-$C_{10}$-Alkyl, $C_3$-$C_7$-Cycloalkyl oder Benzyl ist, und ihre pharmazeutisch verträglichen Additionssalze mit schwachen organischen Säuren sowie ein Verfahren zu ihrer Herstellung.

Die Verbindungen der Formel (I) sind Thromboxansynthetase-inhibitoren.

EP 0 507 763 A1

Die Erfindung betrifft neue therapeutisch wertvolle Thiophen-2-carbonsäurederivate und ein Verfahren zu deren Herstellung.

In der EP-A1-0 109 381 ist eine Verbindung der Formel (I′)

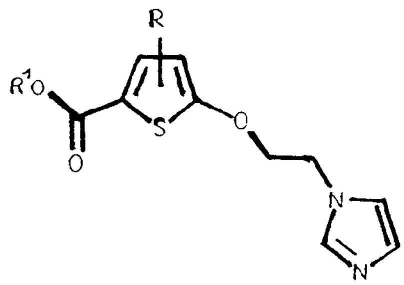

(I′) ,

worin R in Stellung 3 oder 4 Wasserstoff, Methyl, Chlor oder Brom und $R^1$ Wasserstoff oder $C_1$-$C_4$-Alkyl bedeuten, mit starker Hemmwirkung auf die Thromboxansynthetase ohne signifikante Hemmung der Wirkung der Prostacyclinsynthetase- oder Cyclooxygenaseenzyme aus Thrombozytenmikrosomen geoffenbart.

Die Verbindung der Formel (I′), worin $R^1$ H bedeutet, weist bei oraler Verabreichung jedoch nur geringe Resorption auf.

Es wurde nun gefunden, daß die 1-Alkoxycarbonyloxyethylester der Verbindung der Formel (I′) eine verbesserte Resorption bei oraler Verabreichung aufweisen, da sie nach Passage durch den Darm im Blut als freie Carbonsäure vorliegen und daher geeignete Arzneimittelvorläufer ("Prodrugs") der Verbindung der Formel (I′) darstellen.

Die erfindungsgemäßen Verbindungen der im nachstehenden angegebenen Formel (I) wirken dadurch bei oraler Verabreichung auf die Thromboxansynthetase stark hemmend ohne signifikante Hemmung der Wirkung der Prostacyclinsynthetase- oder Cyclooxygenaseenzyme aus Thrombozytenmikrosomen, d.h. diese Verbindungen hemmen die Umwandlung von Prostaglandin-$H_2$ zu Thromboxan-$B_2$ über Thromboxan $A_2$, das ein instabiles Zwischenprodukt ist und das bekanntlich die irreversible Plättchenaggregation induziert und glatte Muskeln, insbesondere die der Blutgefäße, kontrahiert. Diese Tatsache zeigt, daß die Verbindungen der Formel (I) die Biosynthese von Thromboxan $A_2$ hemmen und demgemaß zur Behandlung von Krankheiten geeignet sind, die durch Thromboxan $A_2$ verursacht werden, wie z.B. Entzündung, Hypertonie, Thrombus, Gehirnschlag, Asthma, Angina pectoris, ischämische Herzleiden, ischämische Anfälle, Migräne und vaskuläre Komplikationen von Diabetes.

Gegenstand der vorliegenden Erfindung sind somit neue Verbindungen der allgemeinen Formel (I)

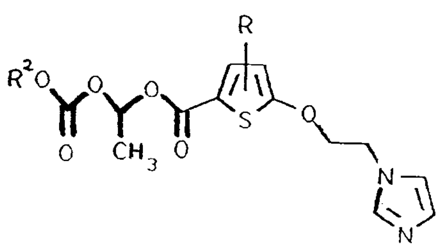

(I) ,

worin R in Stellung 3 oder 4 Wasserstoff, Methyl, Chlor oder Brom darstellt und $R^2$ $C_1$-$C_{10}$-Alkyl, $C_3$-$C_7$-Cycloalkyl oder Benzyl ist, und ihre pharmazeutisch vertraglichen Additionssalze mit schwachen organischen Sauren.

Die Resorption der erfindungsgemäßen Verbindungen bei oraler Verabreichung betragt mindestens das dreifache der Resorption bei oraler Verabreichung der Verbindungen gemäß EP-A1-0 109 381.

Ein weiterer Gegenstand der vorliegenden Erfindung ist ein Verfahren zur Herstellung der neuen Verbindungen der Formel (I), worin R und $R^2$ die oben angegebenen Bedeutungen haben, das darin besteht, daß ein Salz einer Verbindung der Formel (I″)

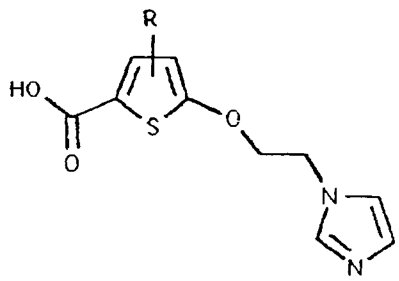

(I") ,

worin R die oben angegebene Bedeutung hat, mit einer Verbindung der allgemeinen Formel (II)

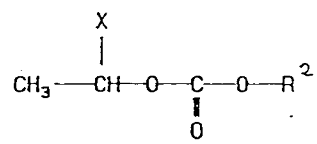

(II) ,

worin X eine zum nukleophilen Austausch geeignete Abgangsgruppe, wie z.B. Halogen, vorzugsweise Chlor oder Brom, darstellt und $R^2$ die oben angegebene Bedeutung hat, umgesetzt wird und die erhaltene Verbindung der Formel (I) in ein Additionssalz mit einer schwachen organischen Säure übergeführt wird.

Die Reaktion wird üblicherweise durch Zugabe von mindestens 1 Äquivalent einer starken Base, wie z.B. eines Alkalihydrids oder Alkalicarbonats, zu einer Lösung der Ausgangsverbindung in einem wasserfreien inerten organischen aprotischen Lösungsmittel, wie z.B. Hexamethylphosphorsäuretriamid, Dimethylformamid oder Dimethylsulfoxid, und Zusetzen der Verbindung der Formel (II), vorzugsweise in äquivalenten Mengen oder in geringem Überschuß im gleichen Lösungsmittel, durchgeführt.

Die Reaktion wird bei einer Temperatur im Bereich von Raumtemperatur bis etwa 100°C durchgeführt. Im allgemeinen wird bevorzugt, das Reaktionsgemisch zu erwärmen, z.B. auf 80°C, um die Reaktion zu beschleunigen. Unter diesen Bedingungen ist die Reaktion üblicherweise innerhalb von 2 ½ Stunden abgeschlossen.

Das Reaktionsgemisch wird auf herkömmliche Weise, beispielsweise durch Lösungsmittelextraktion, aufgearbeitet.

Die erfindungsgemäßen Verbindungen der Formel (I) mit einem basischen Imidazolrest können auf übliche Weise in ihre pharmazeutisch annehmbaren Salze mit schwachen organischen Säuren übergeführt werden. Geeignete Säuren sind beispielsweise Fumar-, Oxal-, Malon-, Bernstein-, Adipin-, Malein-, Wein- oder Citronensäure.

Die Herstellung der Ausgangsverbindung wird in der EP-A1-109 381 beschrieben.

Weiters bezieht sich die vorliegende Erfindung auch auf die Anwendung der neuen Verbindungen der Formel (I) allein oder in Mischung mit anderen Wirksubstanzen in Form üblicher oraler galenischer Zusammensetzungen. Die erfindungsgemaßen Verbindungen können in Form von Tabletten oder Kapseln, welche eine Einheitsdosierung der Verbindung zusammen mit Verdünnungsmitteln, wie Maisstärke, Kalziumcarbonat, Dikalziumphosphat, Alginsaure, Lactose, Magnesiumstearat, Primogel oder Talkum enthalten, oral appliziert werden. Die Tabletten werden in herkömmlicher Weise durch Granulieren der Bestandteile und Pressen und die Kapseln durch Einfüllen in Hartgelatinekapseln geeigneter Größe hergestellt.

Für die orale Applikation beim Menschen wird angenommen, daß der tägliche Dosierungswert einer erfindungsgemäßen Verbindung im Bereich von 0,1 bis 20 mg/kg pro Tag für einen typischen erwachsenen Patienten von 70 kg liegt. Daher können Tabletten oder Kapseln üblicherweise 5 bis 150 mg der aktiven Verbindung für die orale Applikation bis zu dreimal am Tag enthalten.

Selbstverständlich wird aber der Arzt in jedem Fall die tatsächliche Dosierung bestimmen, welche für den individuellen Patienten am geeignetsten ist, wobei diese mit dem Alter, der Masse und dem Ansprechen des Patienten variieren kann.

Das folgende Beispiel soll die Erfindung erläutern, ohne sie jedoch einzuschränken.

**Beispiel 1:**

Einer Lösung von 5 g (18,20 mMol) 5-[2-(1H-Imidazol-1-yl)-ethoxy]-thiophen-2-carbonsäurehydrochlorid in 100 ml Hexamethylphosphorsäuretriamid (HMPT) werden bei Raumtemperatur unter gutem Rühren 2 g NaH (80 %ige Suspension) portionsweise zugegeben. Dabei steigt die Temperatur auf etwa 40°C an. Zur Salzbildung wird 1 h bei Raumtemperatur gerührt und anschließend eine Lösung von 4,2 g (27,52 mMol) 1-Chlorethylethylcarbonat in 6 ml HMPT bei Raumtemperatur zugetropft.

Das Reaktionsgemisch wird 2 ½ h auf 80°C erhitzt und anschließend zwischen Eiswasser und Ethylacetat (EtOAc) verteilt. Die Phasen werden getrennt, die wässerige Phase dreimal mit EtOAc ausgeschüttelt und die organische Phase zweimal mit einer gesättigten $NaHCO_3$-Lösung extrahiert. Die EtOAc-Phase wird dreimal mit 2n HCl ausgeschüttelt; die HCl-Phase wird unter Eiskühlung neutralisiert und anschließend mit EtOAc erschöpfend extrahiert.

Nach dem Trocknen über $Na_2SO_4$ wird filtriert und eingedampft. Es werden 5,78 g gelbes Öl erhalten, das säulenchromatographisch gereinigt wird: Kieselgel, $CH_2Cl_2$/Ethanol = 20:1. Es werden 4,43 g 5-[2-(1H-Imidazol-1-yl)-ethoxy]-thiophen-2-carbonsäure-1-ethoxycarbonyloxyethylester (68,7 % der Theorie) als blaßgelbes Öl erhalten.

Zur Bildung des Fumarats wird das Öl in wenig Ethanol p.A. gelöst und bei -8°C mit der äquimolaren Menge Fumarsäure (gelöst in Ethanol/Methanol = 6:1) versetzt. Nach mehrstündigem Rühren unter Eiswasserkühlung wird schonend eingedampft und der Rückstand mit eiskaltem Ether zur Kristallisation gebracht. Das erhaltene Fumarat wird aus EtOAc umkristallisiert, wobei 4,0 g 5-[2-(1H-Imidazol-1-yl)-ethoxy]-thiophen-2-carbonsäure-1-ethoxycarbonyloxyethylesterfumarat (62,0 % der Theorie), Fp. 73-75°C, als farblose Kristalle erhalten werden. DC: $CH_2Cl_2$/Ethanol = 12:1.

Mikroelementaranalyse:

| | |
|---|---|
| Berechnet für $C_{19}H_{22}N_2O_{12}S$: | C 48,51; H 4,71; N 5,95 %; |
| gefunden: | C 48,49; H 4,59; N 5,97 %; |

MG = 470,46

$^1$H-NMR (DMSO): 7,50 (s, 1H, Im-$H_2$); 7,36; 7,31; 6,11; 6,06 (AB, 2H, Th-$H_3$ und Th-$H_4$); 6,87 (s, breit, 2H, Im-$H_4$ und Im-$H_5$); 6,68 (q, 1H, -CH-$CH_3$); 6,55 (s, 2H, -CH=CH-); 4,18 (h, 4H, -$OCH_2CH_2$-); 3,98 (q, 2H, -$OCH_2$-$CH_3$); 1,37 (d, 3H, $CH_3$-CH-); 1,08 (t, 3H, -$OCH_2$-$CH_3$).

## Patentansprüche

1. Verbindungen der allgemeinen Formel (I)

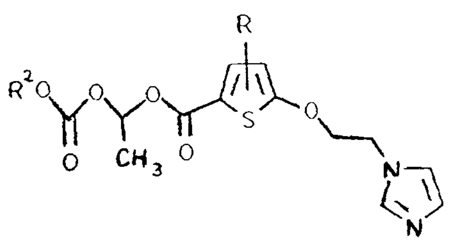

(I) ,

worin R in Stellung 3 oder 4 Wasserstoff, Methyl, Chlor oder Brom darstellt und $R^2$ $C_1$-$C_{10}$-Alkyl, $C_3$-$C_7$-Cycloalkyl oder Benzyl ist, und ihre pharmazeutisch verträglichen Additionssalze mit schwachen organischen Säuren.

2. 5-[2-(1H-Imidazol-1-yl)-ethoxy]-thiophen-2-carbonsäure-1-ethoxycarbonyloxyethylester.

3. 5-[2-(1H-Imidazol-1-yl)-ethoxy]-thiophen-2-carbonsäure-1-ethoxycarbonyloxyethylesterfumarat.

4. Verfahren zur Herstellung einer Verbindung nach Anspruch 1, dadurch gekennzeichnet, daß ein Salz einer Verbindung der Formel (I″)

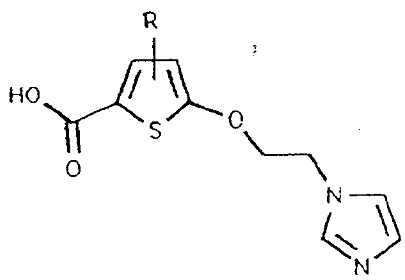

(I") ,

worin R die oben angegebene Bedeutung hat, mit einer Verbindung der allgemeinen Formel (II)

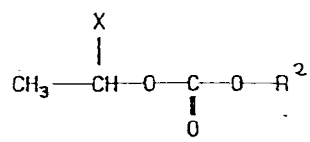

(II) ,

worin X eine zum nukleophilen Austausch geeignete Abgangsgruppe, wie z.B. Halogen, darstellt und $R^2$ die oben angegebene Bedeutung hat, umgesetzt wird und die erhaltene Verbindung der Formel (I) in ein Additionssalz mit einer schwachen organischen Säure übergeführt wird.

5. Verfahren nach Anspruch 4, dadurch gekennzeichnet, daß man eine Verbindung (II) einsetzt, worin X Chlor oder Brom ist.

6. Pharmazeutische Zusammensetzung zur oralen Verabreichung, dadurch gekennzeichnet, daß sie eine Verbindung der Formel (I), wie in Anspruch 1 definiert, oder ein pharmazeutisch annehmbares Additionssalz hievon mit einer schwachen organischen Säure zusammen mit einem pharmazeutisch annehmbaren Träger oder Verdünnungsmittel enthält.

7. Verwendung einer Verbindung der Formel (I), wie in Anspruch 1 definiert, als Inhibitor von Thromboxansynthetase.

8. Verwendung einer Verbindung der Formel (I), wie in Anspruch 1 definiert, zur Herstellung von Medikamenten zur Behandlung von durch Thromboxan $A_2$ verursachten Krankheiten, wie beispielsweise Entzündung, Hypertonie, Thrombus, Gehirnschlag, Asthma, Angina pectoris, ischämische Herzleiden, ischämische Anfälle, Migräne und vaskuläre Komplikationen von Diabetes.

Europäisches Patentamt

# EUROPÄISCHER RECHERCHENBERICHT

Nummer der Anmeldung

EP 92 89 0075

| EINSCHLÄGIGE DOKUMENTE | | | | |
|---|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) | RECHERCHIERTE SACHGEBIETE (Int. Cl.5 ) |
| D,A | EP-A-0 109 381 (LAEVOSAN G.M.B.H. & CO KG.)<br>* Zusammenfassung und Ansprüche *<br>--- | 1,6-8 | C07D409/12<br>A61K31/41 | C07D<br>A61K |
| A | GB-A-2 065 121 (PFIZER LTD.)<br>* Zusammenfassung, Beispiel 21 und Ansprüche *<br>--- | 1,6-8 | | |
| A | DE-A-2 126 597 (FARBWERKE HOECHST AG)<br>* Ansprüche *<br>----- | 1,4-8 | | |

Der vorliegende Recherchenbericht wurde für alle Patentansprüche erstellt

| Recherchenort | Abschlußdatum der Recherche | Prüfer |
|---|---|---|
| DEN HAAG | 23 JUNI 1992 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)